# EUROPÄISCHE PATENTSCHRIFT

(11) **EP 0 907 645 B1**
(45) Veröffentlichungstag und Bekanntmachung des Hinweises auf die Patenterteilung: **13.02.2002**
(21) Anmeldenummer: 97927174.9
(22) Anmeldetag: 12.06.1997
(51) Int. Cl.: C07D 307/88

(54) **VERFAHREN ZUR REINIGUNG VON PHTHALID**
PROCESS FOR PURIFYING PHTHALIDE
PROCEDE DE PURIFICATION DE PHTHALIDE

(30) Priorität: 27.06.1996 DE 19625693
(43) Veröffentlichungstag der Anmeldung: 14.04.1999
(73) Patentinhaber: BASF AKTIENGESELLSCHAFT, 67056 Ludwigshafen (DE)
(72) Erfinder: REIF, Wolfgang, D-67227 Frankenthal (DE); MASSONNE, Klemens, D-67368 Westheim (DE); NEUHAUSER, Horst, D-67373 Dudenhofen (DE)
(74) Vertreter: Fitzner, Uwe, Dr.
(86) Internationale Anmeldenummer: EP9703045
(87) Internationale Veröffentlichungsnummer: WO9800417

(56) Entgegenhaltungen:
- DE-A- 2 720 929
- DE-A- 2 803 319
- DE-A- 3 245 544

## Beschreibung

Die vorliegende Erfindung betrifft ein Verfahren zur Reinigung eines durch Carbonsäuren, Carbonsäureanhydride und/oder Carbonsäureester verunreinigten Phthalids.

Für die Herstellung von Phthalid sind verschiedene Synthesen ausgehend von Phthalsäure-Derivaten beschrieben. In der Regel wird Phthalid durch die selektive Hydrierung von Phthalsäureanhydrid hergestellt (DE-C-28 03 319).

Problematisch ist in der Regel die Aufarbeitung und Reinigung der bei der Herstellung erhaltenen Reaktionsprodukte, da bei der Phthalidsynthese fast zwangsläufig ein Rohprodukt entsteht, das durch Carbonsäuren und deren Derivate wie Phthalsäure, Phthalsäureanhydrid, Phthalsäuremonomethylester, Tetrahydrophthalid und 2-Methylbenzoesäure verunreinigt ist. Die Abtrennung dieser Nebenprodukte ist sehr aufwendig und teilweise rein destillativ nicht möglich.

Es ist bekannt, daß sich besonders Phthalsäureanhydrid und Phthalsäuremonomethylester destillativ von Phthalid nicht vollständig abtrennen lassen. Enthält das Rohprodukt Phthalsäure, wird daraus zusätzliches Phthalsäureanhydrid während der Destillation gebildet.

Die Probleme bei der Abtrennung von Nebenprodukten führen dazu, daß bei der destillativen Aufarbeitung Phthalid als Wertprodukt in nur unbefriedigender Ausbeute und Reinheit erhalten wird.

Es ist bereits aus DE-C-32 45 544 ein Verfahren zur destillativen Reinigung von durch Carbonsäuren verunreinigtem Phthalid bekannt, bei dem man das zu reinigende Phthalid vor der Destillation mit n-Butanol oder iso-Butanol versetzt und mehrere Stunden in einem Autoklaven auf Temperaturen zwischen 150 und 280°C erhitzt, bevor es anschließend einer fraktionierten Destillation unterworfen wird. Die Nachteile dieser Verfahrensweise sind allerdings, daß das Verfahren sehr zeitaufwendig ist und das überschüssige Butanol vor der Destillation des Phthalids abdestilliert werden muß.

Aufgabe der vorliegenden Erfindung war daher ein einfaches und verbessertes Verfahren zur Reinigung von im wesentlichen durch Carbonsäuren und deren Derivate verunreinigtem Phthalid.

Es wurde nun ein vorteilhaftes Verfahren zur Reinigung von Phthalid, das durch Carbonsäuren, Carbonsäureanhydride und/oder Carbonsäureester verunreinigt ist, gefunden, welches dadurch gekennzeichnet ist, daß man zu dem zu reinigenden Phthalid Polyethylenglykol und/oder Polypropylenglykol hinzufügt und anschließend fraktionierend destilliert.

Im allgemeinen wird zu dem zu reinigenden Phthalid das Polyethylenglykol und/oder Polypropylenglykol in Mengen von 1 bis 50 Gew.-%, zweckmäßig von 2 bis 40 Gew.-%, vorzugsweise von 3 bis 30 Gew.-%, insbesondere von 5 bis 15 Gew.-%, besonders vorteilhaft von 8 bis 12 Gew.-%, bezogen auf Phthalid, hinzugefügt.

Zweckmäßig verwendet man Polyethylenglykol und Polypropylenglykol, deren mittlere Molmasse im Bereich von 200 bis 600, vorzugsweise im Bereich von 250 bis 400 liegen.

Die Polyethylenglykole werden zweckmäßig erhalten durch Polyaddition von Ethylenoxid in meist geringe Mengen Wasser enthaltenden Systemen mit Ethylenglykol als Startmolekül. Die Polypropylenglykole entstehen entsprechend durch Polyaddition von Propylenoxid mit 1,2-Propandiol als Startmolekül.

Es ist ein Vorteil des erfindungsgemäßen Verfahrens, daß in der Regel schon eine relativ geringe Zugabe von Polyethylenglykol bzw. Polypropylenglykol zum Phthalid-Rohprodukt für das Reinigungsverfahren ausreicht. Es ist ein weiterer Vorteil des Verfahrens, daß auf eine zeitlich aufwendige thermische Vorbehandlung des mit dem Polyethylenglykol bzw. Polypropylenglykol versetzten Phthalid-Rohproduktes verzichtet werden kann. Ein vorheriges Erhitzen, z.B. unter Rückfluß oder im Autoklav, ist nicht erforderlich. Dementsprechend besteht eine bevorzugte Ausführungsform des Reinigungsverfahrens darin, daß man direkt nach der Zugabe von Polyethylenglykol oder Polypropylenglykol zum Phthalid-Rohprodukt ohne weitere Vorbehandlung die fraktionierte Destillation anschließt. Wählt man die chemische Struktur und die Molmasse der Hilfskomponente Polyethylenglykol bzw. Polypropylenglykol in der Weise aus, daß die Hilfskomponente keine niedriger als Phthalid siedenden Anteile enthält, ist Phthalid einer der Leichtsieder im Destillationssystem. Bei Anwendung dieser bevorzugten Arbeitsweise bleiben unumgesetztes Polyethylenglykol bzw. Polypropylenglykol und deren Umsetzungsprodukte im Sumpf der fraktionierten Destillation.

Das nach dem erfindungsgemäßen Verfahren erhaltene Phthalid zeichnet sich durch besonders gute Reinheit aus und wird gleichzeitig in guter Ausbeute erhalten, wobei die Ausbeute auch gegenüber einer Destillation ohne Zusatz der Hilfskomponenten Polyethylenglykol bzw. Polypropylenglykol höher ist. Das im Sumpf verbleibende Polyethylenglykol bzw. Polypropylenglykol trägt außerdem dazu bei, den Sumpfrückstand flüssig zu halten. Dies ist vorteilhaft einerseits für die Entfernung des Destillationsrückstands aus der Destillationsblase und andererseits für eine bevorzugte Arbeitsweise, bei der die Destillationsblase mehrmalig nach jeweils beendeter fraktionierter Destillation des zu reinigenden Rohphthalids erneut mit Rohphthalid gefüllt wird und dann fraktionierend destilliert wird, ohne daß jeweils der Destillationsrückstand aus der vorhergehenden fraktionierten Destillation entfernt wird.

Phthalid ist ein wichtiges Zwischenprodukt beispielsweise für die Herstellung von Pflanzenschutzmitteln.

Die folgenden Beispiele erläutern die Erfindung.

### Beispiel 1

Für die fraktionierte Destillation wurden 1600 g einer Phthalid-Rohlösung eingesetzt, die etwa folgende Zusammensetzung hatte: 46,0 Gew.-% Butyrolacton, 34,9 % Phthalid, 15,0 Gew.-% Wasser, 2,5 Gew.-% 2-Methylbenzoesäure, 1,0 Gew.-% Phthalsäureanhydrid, 0,3 Gew.-% Tetrahydrophthalid und 0,3 Gew.-% weitere Nebenprodukte. Zu der Phthalid-Rohlösung wurden 176 g (32 Gew.-% bezogen auf Phthalid) eines Polyethylenglykols mit einer mittleren molaren Masse von 300 gegeben. Anschließend wurde ohne eine weitere Vorbehandlung über eine Laborkolonne mit 40 theoretischen Trennstufen bei 20 mbar Kopf druck fraktionierend destilliert.
Die Destillationsausbeute betrug 73 % Phthalid in einer Reinheit von mehr als 98,5 %.

Der Destillationsrückstand wurde anschließend nicht zur Entsorgung entfernt, sondern verblieb in der Destillationsblase. Durch mehrmaliges Füllen der Blase mit weiterer Phthalid-Rohlösung und jeweiliger Destillation wurde die Phthalidgesamtausbeute auf 87 % gesteigert. Der Phthalsäureanhydrid-Gehalt im Phthalid-Reinprodukt lag unter 0,1 %, der 2-Methylbenzoesäure-Gehalt lag unter 0,2 %.

### Beispiel 2

Die fraktionierte Destillation wurde analog zu Beispiel 1 durchgeführt, wobei jedoch der Phthalid-Rohlösung vor der Destillation Polyethylenglykol mit der mittleren molaren Masse von 300 lediglich in einer Menge von 9 Gew.-%, bezogen auf Phthalid, zugegeben wurde. Es wurde Phthalid in einer Ausbeute von 87 % mit einer Reinheit von mehr als 98,5 % erhalten, wobei der Phthalsäureanhydrid-Gehalt unter 0,1 % und der 2-Methylbenzoesäure-Gehalt unter 0,3 % lag.

### Vergleichsbeispiel (nicht erfindungsgemäß)

Für die fraktionierte Destillation wurden 1600 g einer Phthalid-Rohlösung der gleichen Zusammensetzung wie in Beispiel 1 eingesetzt. Die Phthalid-Rohlösung wurde über eine Laborkolonne mit 40 theoretischen Trennstufen bei 20 mbar Kopfdruck destilliert. Die Destillationsausbeute betrug 58 % Phthalid, das in einer Reinheit von 97,6 % und mit einem Phthalsäureanhydrid-Gehalt von 1,0 % anfiel. Für die meisten Anwendungen kann eine Phthalidware dieser Reinheit nicht eingesetzt werden.

## Patentansprüche

1. Verfahren zur Reinigung von Phthalid, das durch Carbonsäuren, Carbonsäureanhydride und/oder Carbonsäureester verunreinigt ist, **dadurch gekennzeichnet, daß** man zu dem zu reinigenden Phthalid Polyethylenglykol und/oder Polypropylenglykol hinzufügt und anschließend fraktionierend destilliert.

2. Verfahren nach Anspruch 1, **dadurch gekennzeichnet, daß** man zu dem zu reinigenden Phthalid Polyethylenglykol und/oder Polypropylenglykol hinzufügt, dessen mittlere Molmasse im Bereich von 200 bis 600 liegt.

3. Verfahren nach Anspruch 1, **dadurch gekennzeichnet, daß** man zu dem zu reinigenden Phthalid Polyethylenglykol und/oder Polypropylenglykol hinzufügt, dessen mittlere Molmasse im Bereich von 250 bis 400 liegt.

4. Verfahren nach Anspruch 1 bis 3, **dadurch gekennzeichnet, daß** man zu dem zu reinigenden Phthalid 1 bis 50 Gew.-%, bezogen auf Phthalid, des Polyethylenglykols und/oder PolypropylenGlykols hinzufügt.

5. Verfahren nach Anspruch 1-4, **dadurch gekennzeichnet, daß** man 2-40 Gew.-% Polyethylenglykol hinzufügt.

6. Verfahren nach Anspruch 1 bis 4, **dadurch gekennzeichnet, daß** man zu dem zu reinigenden Phthalid Polyethylenglykol und/oder Polypropylenglykol hinzufügt und anschließend direkt ohne weitere Vorbehandlung fraktionierend destilliert.

7. Verfahren nach einem der Ansprüche 1-6, **dadurch gekennzeichnet, daß** das hinzugefügte Polyethylenglycol und/oder Polypropylenglykol keine niedriger als Phthalid siedenden Anteile enthält.

8. Verfahren nach einem der Ansprüche 1-7, **dadurch gekennzeichnet, daß** unumgesetztes Polyethylenglykol und/oder Polypropylenglykol im Sumpf der fraktionierten Destillation verbleiben und Phthalid als Leichtsieder abdestilliert wird.

9. Verfahren nach Anspruch 8, **dadurch gekennzeichnet, daß** der Sumpfrückstand nach jeweils beendeter Destillation in der Destillationsblase verbleibt und die Destillationsblase erneut mit Rohphthalid gefüllt wird.

10. Verfahren nach einem der Anspruche 1-9, wobei Phthalid in einer Reinheit von mehr als 98,5% erhalten wird.

## Claims

1. A process for purifying phthalide contaminated by carboxylic acids, carboxylic anhydrides and/or carboxylic esters, which comprises adding polyethylene glycol and/or polypropylene glycol to the phthalide to be purified and then subjecting the mixture to a fractional distillation.

2. A process as claimed in claim 1, wherein the average molar mass of the polyethylene glycol and/or polypropylene glycol added to the phthalide to be purified is within the range from 200 to 600.

3. A process as claimed in claim 1, wherein the average molar mass of the polyethylene glycol and/or polypropylene glycol added to the phthalide to be purified is within the range from 250 to 400.

4. A process as claimed in any of claims 1 to 3, wherein the amount of polyethylene glycol and/or polypropylene glycol added to the phthalide to be purified is within the range from 1 to 50% by weight, based on phthalide.

5. A process as claimed in any of claims 1 to 4, wherein 2-40% by weight of polyethylene glycol is added.

6. A process as claimed in any of claims 1 to 4, wherein polyethylene glycol and/or polypropylene glycol are added to the phthalide to be purified and then a fractional distillation is carried out directly without further pretreatment.

7. A process as claimed in any of claims 1 to 6, wherein the added polyethylene glycol and/or polypropylene glycol includes no fractions having a boiling point lower than phthalide.

8. A process as claimed in any of claims 1 to 7, wherein unconverted polyethylene glycol and/or polypropylene glycol stay in the bottom product of the fractional distillation and phthalide is distilled off as a low boiler.

9. A process as claimed in claim 8, wherein the bottom product residue after each distillation remains in the distillation flask and the distillation flask is recharged with crude phthalide.

10. A process as claimed in any of claims 1 to 9, wherein phthalide is obtained in a purity of more than 98.5%.

## Revendications

1. Procédé de purification de phtalide contaminé par des acides carboxyliques, des anhydrides carboxyliques et/ou des esters carboxyliques, **caractérisé en ce que** l'on ajoute du polyéthylèneglycol et/ou du polypropylèneglycol au phtalide destiné à être purifié et on procède ensuite à une distillation fractionnée.

2. Procédé selon la revendication 1, **caractérisé en ce que** l'on ajoute, au phtalide destiné à être purifié, du polyéthylèneglycol et/ou du polypropylèneglycol dont la masse molaire moyenne est dans la gamme de 200 à 600.

3. Procédé selon la revendication 1, **caractérisé en ce que** l'on ajoute, au phtalide destiné à être purifié, du polyéthylèneglycol et/ou du polypropylèneglycol dont la masse molaire moyenne est dans la gamme de 250 à 400.

4. Procédé selon les revendications 1 à 3, **caractérisé en ce que** l'on ajoute au phtalide destiné à être purifié, de 1 à 50% en poids, par rapport au phtalide, du polyéthylèneglycol et/ou polypropylèneglycol.

5. Procédé selon les revendications 1 à 4, **caractérisé en ce que** l'on ajoute de 2 à 4% pour cent en poids de polypropylèneglycol.

6. Procédé selon les revendications 1 à 4, **caractérisé en ce que** l'on ajoute du polyéthylèneglycol et/ou du polypropylèneglycol au phtalide destiné à être purifié et on procède ensuite directement à une distillation fractionnée sans prétraitement supplémentaire.

7. Procédé selon l'une quelconque des revendications 1 à 6, **caractérisé en ce que** le polyéthylèneglycol et/ou polypropylèneglycol ajoutés ne comprennent aucune fraction ayant un point d'ébullition inférieur à celui du phtalide.

8. Procédé selon l'une quelconque des revendications 1 à 7, **caractérisé en ce que** le polyéthylèneglycol et/ou polypropylèneglycol n'ayant par réagi restent en queue de la distillation fractionnée et le phtalide est distillé en tant que fraction à faible point d'ébullition.

9. Procédé selon la revendication 8, **caractérisé en ce que** le résidu de queue reste dans le vase de distillation à la fin de chaque distillation et le vase de distillation est de nouveau rempli de phtalide brut.

10. Procédé selon l'une quelconque des revendications 1 à 9, dans lequel le phtalide obtenu a une pureté supérieure à 98,5%.
